# EUROPEAN PATENT SPECIFICATION

(11) **EP 2 758 550 B1**
(45) Date of publication and mention of the grant of the patent: **26.10.2016**
(21) Application number: 12778840.4
(22) Date of filing: 24.09.2012
(51) Int. Cl.: C12Q 1/68

(54) **DETECTION OF ISOTYPE PROFILES AS SIGNATURES FOR DISEASE**
NACHWEIS VON ISOTYPENPROFILEN ALS ANZEICHEN FÜR ERKRANKUNGEN
DÉTECTION DE PROFILS D'ISOTYPES COMME SIGNATURES D'UNE MALADIE

(30) Priority: 22.09.2011 US 201161537878 P
(43) Date of publication of application: 30.07.2014
(73) Proprietor: Lineage Biosciences, Inc., Palo Alto, CA 94304 (US)
(72) Inventor: HUTCHINS, Maria, U., Mountain View, CA 94043 (US); SELIGSON, Daniel, Palo Alto, CA 94301 (US)
(74) Representative: Patent Boutique LLP
(86) International application number: PCT/US2012/056911
(87) International publication number: WO 2013/044234

(56) References cited:
- CAMERON L A ET AL: "Expression of IL-4, Cepsilon RNA, and Iepsilon RNA in the nasal mucosa of patients with seasonal rhinitis: Effect of topical corticosteroids", JOURNAL OF ALLERGY AND CLINICAL IMMUNOLOGY, MOSBY, INC, US, vol. 101, no. 3, 1 March 1998 (1998-03-01) , pages 330-336, XP027420966, ISSN: 0091-6749, DOI: 10.1016/S0091-6749(98)70244-1 [retrieved on 1998-03-01]
- WHITE H N: "Restriction-PCR fingerprinting of the immunoglobulin VH repertoire: Direct detection of an immune response and global analysis of B cell clonality", EUROPEAN JOURNAL OF IMMUNOLOGY, WILEY - V C H VERLAG GMBH & CO. KGAA, DE, vol. 28, no. 10, 1 October 1998 (1998-10-01), pages 3268-3279, XP002281079, ISSN: 0014-2980, DOI: 10.1002/(SICI)1521-4141(199810)28:10<3268: :AID-IMMU3268>3.3.CO;2-6
- WECKERT H A ET AL: "Quantifiable analysis of human immunoglobulin heavy chain class-switch recombination to all isotypes", JOURNAL OF IMMUNOLOGICAL METHODS, ELSEVIER SCIENCE PUBLISHERS B.V.,AMSTERDAM, NL, vol. 233, no. 1-2, 1 January 2000 (2000-01-01), pages 141-158, XP004188253, ISSN: 0022-1759, DOI: 10.1016/S0022-1759(99)00132-5
- HONG-YING WANG ET AL: "A custom 148 gene-based resequencing chip and the SNP explorer software: new tools to study antibody deficiency", HUMAN MUTATION, vol. 31, no. 9, 22 July 2010 (2010-07-22), pages 1080-1088, XP055045331, ISSN: 1059-7794, DOI: 10.1002/humu.21322
- CHEUNG S C ET AL: "A RECOMBINANT HUMAN FAB EXPRESSED IN ESCHERICHIA COLI NEUTRALIZES RABIES VIRUS", JOURNAL OF VIROLOGY, THE AMERICAN SOCIETY FOR MICROBIOLOGY, US, vol. 66, no. 11, 1 November 1992 (1992-11-01), pages 6714-6720, XP001031105, ISSN: 0022-538X
- WILLIAMS: "Rheumatoid factor isotype switch and somatic mutation variants within rheumatoid arthritis synoviu", IMMUNOLOGY, vol. 98, 1 January 1999 (1999-01-01), pages 123-136, XP055045332,

## Description

### Field of the Invention

The present invention relates to the field of quantitative nucleic acid analysis. More specifically, the present invention provides a non-invasive technique for the detection and quantitation of immunoglobulin isotypes present in a biological sample, and the generation of specific isotype profiles as signatures for disease.

### Background

The immune system comprises the innate and the adaptive immunity systems. The innate immune system comprises the cells and mechanisms utilizing generic methods to recognize foreign pathogens. Cells involved in innate immunity include neutrophils, natural killer cells, macrophages, monocytes, basophils, eosinophils, mast, and dendritic cells. These cells carry out the act of phagocytosis as well as the release of many chemicals that kill invading pathogens. In addition, these cells are involved in innate immunity defense mechanisms including the complement cascade and inflammation. Finally, some of these cells participate in the antigen presentation process that plays a role in the adaptive immunity system.

The adaptive immunity system has evolved to attack specific features on their targets. The occurrence of one response to a specific target provides the host with "memory" of it, causing it to mount a stronger response if it were to appear another time. Usually any protein or polysaccharide can serve as the target for some subset of the adaptive immune response cells or their products that recognize specific epitopes on the target. The adaptive immune response is divided into two types: the humoral and the cell-mediated immune response, and B-cells and T-cells play the specificity roles in these responses, respectively.

Since autoimmune disease involves the recognition of some element of the adaptive immune system to self targets, aspects of the adaptive immune system have been examined to aid in diagnosis and prognosis. Using standard immunological techniques, the humoral immune system has been investigated by looking for circulating autoantibodies. Autoantibodies, like antinuclear, anti-dsDNA, and rheumatoid factor, have been identified for several diseases. These antibodies may not themselves be pathological, nor is the target they recognize in the body necessarily the same as that tested for in vitro; however, measurement of their levels aids in the diagnosis and in some cases has some prognostic and treatment implications.

Another methodology to study the adaptive immune system in autoimmune disease is based on the analysis of the diversity of the adaptive immune cells. Activation of the adaptive immune cells leads to their clonal expansion. Evidence of this clonal expansion is usually achieved by amplification from the blood RNA or DNA of part of the nucleic acid sequence coding for the antigen recognition region. For example, PCR primers to amplify sequences that have a specific V segment of the β chain in T-cell receptor (analogous to antibody heavy chain) are used to amplify the J segments or J and D segments connected to the specific V segment. When a diverse cell population is present it is expected to amplify fragments with a distribution of slightly different size amplicons, but clonal expansion causes specific sizes to become enriched and thus more intense as visualized as bands on a gel. In the technique called spectratyping each of the V segments is amplified with the J and D segments to assess whether any of these amplicons shows a clonal expansion.

One problem of the spectratyping approach is that many distinct sequences can have the same length and hence are indistinguishable. Therefore only dramatic clonal expansion can be discerned by this technique. There is a need to improve methods of diagnosing and aiding prognosis of autoimmune disease and autoimmune disease states as well as other diseases for which the immune system plays a central role.

The vast diversity of the immune system provides it with an immense reserve of potentially useful cells but also presents a challenge to the researcher trying to use this repertoire for predictive purposes. Any single sequence targeting an antigen is one of a vast number that could be involved with and/or correlated to the disease process in a given individual. Methods that would identify which of the many cells in a given individual are involved with disease processes would be of great value to human health.

### Summary

The present invention provides a method of determining whether an immunoglobulin isotype profile determined from a human subject-derived biological sample is indicative of a disease state, comprising: a. isolating and amplifying a plurality of RNA nucleic acids from the biological sample, wherein the RNA comprises Ig isotype constant region sequences, to generate amplicons that comprise immunoglobulin constant region sequences, wherein the biological sample comprises a plurality of different cell types including at least 10,000 B-cells; b. sequencing the amplicons in a massively parallel sequencing reaction using a sequencing technique that generates at least 10,000 sequence reads per run; c. analyzing sequence data from (b) by extracting Ig constant region sequences characteristic of each isotype, and comparing the number of isotype sequences for each isotype thereby determining the immunoglobulin isotype profile; and d. determining whether the immunoglobulin isotype profile is characteristic of a disease state, wherein the disease state is an autoimmune disease, an infectious disease, or cancer. The present specification provides methods for monitoring the immune repertoire and profiling the immune system. In contrast to methods previously described which specifically require specific populations of immune cells (e.g., T-cells or B-cells) and spatial isolation of the individual cells and/or individual nucleic acid molecules derived from such cells, the methods of the present invention are performed using a heterogeneous population of cells, and a heterogeneous mixture of nucleic acids derived therefrom.

The specification provides a method of determining immunoglobulin isotype in a whole blood sample by isolating a plurality of nucleic acids from a biological sample comprising a plurality of cell types obtained from a subject and detecting sequences specific for the constant regions of immunoglobulin in the plurality of nucleic acids; thereby determining the immunoglobulin isotype.

The method generally involves the steps of obtaining a nucleic acid from a biological sample that includes a plurality of different cell types from a subject, isolating nucleic acid from the biological sample, detecting a sequence specific for one or more regions of immunoglobulin, and determining the different levels of sequences to generate an immunoglobulin isotype profile.

Biological samples having a plurality of different cell types include, but are not limited to, blood, a blood fraction, saliva, sputum, urine, semen, transvaginal fluid, cerebrospinal fluid, stool, a cell and tissue biopsies. In preferred embodiments the sample is whole blood and the sample size is less than 100 µL.

The nucleic acid isolated from such biological samples can be DNA (e.g., cDNA) or RNA. In certain embodiments, the isolated nucleic acid is total RNA. In certain embodiments, the isolated nucleic acid is cDNA generated from total RNA. In some embodiments, the cDNA is amplified using a plurality of primers specific for one or more regions of immunoglobulin, such as the immunoglobulin VDJ region and/or the Ig constant region.

The detection step can be performed using hybrid capture or sequencing techniques. Examples of sequencing techniques useful in the methods of the invention include but are not limited to sequencing-by-synthesis technology, such as massively parallel sequencing, single molecule sequencing, true single molecule sequencing, pyrosequencing, etc. Suitable sequencing platforms that are useful with methods of the invention include, but are not limited to, True Single Molecule Sequencing (tSMS™) technology such as the HeliScope™ Sequencer offered by Helicos Inc., Single Molecule Real Time (SMRT™) technology, such as the PacBio RS system offered by Pacific Biosciences, massively parallel sequencing technology, such as the HiSEQ™ and MiSEQ™ systems offered by Illumina, Inc., the Solexa™ Sequencer offered by Illumina, Inc., the SOLiD™ sequencing system, offered by Life Technologies, Inc., and the Ion Torrent system offered by Life Technologies, Inc.

In a particular instance, the present invention provides methods for profiling the immune system using sequencing techniques to sequence immunoglobulin isotypes directly from nucleic acid derived from peripheral whole blood, or a fraction thereof. In another particular embodiment, immunoglobulin isotype profiles are obtained by direct sequencing from nucleic acid derived from peripheral blood mononuclear cells. As used herein, the term "peripheral whole blood" refers to blood from which no constituent, such as red blood cells, white blood cells, plasma, or platelets, has been removed, and the term "peripheral blood mononuclear cells" or "PBMCs" refers to a mixture of blood cells having a round nucleus, and including lymphocytes, monocytes and macrophages.

The profiles of the immune system generated by the methods of the invention can be used for diagnosis of diseases and disorders, and for diagnosis of states of diseases and disorders. The methods of the invention can be used in monitoring diseases and disorders and assessing treatment of diseases and disorders. The diseases and disorders that the methods of the provided invention can be applied to include autoimmune disease, including systemic lupus erythematosus (SLE), multiple sclerosis (MS), rheumatoid arthritis (RA), and ankylosing spondylitis. The methods of the provided invention can also be applied to the diagnosis, monitoring, and treatment of transplant rejection and immune aging. Furthermore, the methods of immune profiling described herein can be used for diagnosing, monitoring, and treating other diseases related to the immune system, including cancer and infectious disease.

### Brief Description of the Drawings

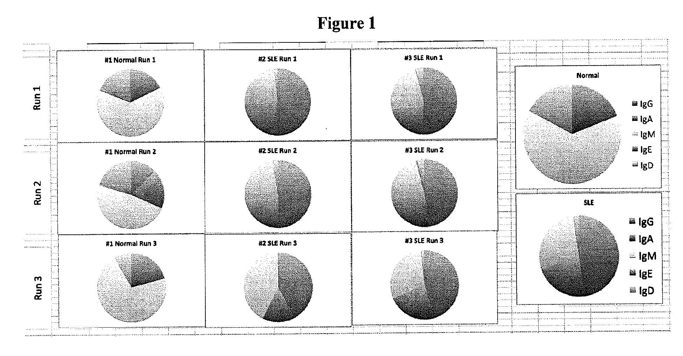

FIG. 1 shows pie charts illustrating the isotype distribution patterns for three samples (#1, #2 and #3) obtained on three different sequencing runs.

### Detailed Description

The present invention provides a method of determining whether an immunoglobulin isotype profile determined from a human subject-derived biological sample is indicative of a disease state, comprising: a. isolating and amplifying a plurality of RNA nucleic acids from the biological sample, wherein the RNA comprises Ig isotype constant region sequences, to generate amplicons that comprise immunoglobulin constant region sequences, wherein the biological sample comprises a plurality of different cell types including at least 10,000 B-cells; b. sequencing the amplicons in a massively parallel sequencing reaction using a sequencing technique that generates at least 10,000 sequence reads per run; c. analyzing sequence data from (b) by extracting Ig constant region sequences characteristic of each isotype, and comparing the number of isotype sequences for each isotype thereby determining the immunoglobulin isotype profile; and d. determining whether the immunoglobulin isotype profile is characteristic of a disease state, wherein the disease state is an autoimmune disease, an infectious disease, or cancer. Methods and materials described herein apply sequencing techniques for analyzing immune receptor gene populations and the immunoglobulin isotype distribution in a biological sample obtained from a subject. Sequencing of the immune receptor gene populations offers specific and detailed molecular characterization as well as high sensitivity for detecting sequences of interest for aiding in the diagnosis and monitoring of disease.

Methods for profiling the immune repertoire utilizing microchip arrays (e.g., ImmunArray), or sequencing techniques have been described. However, such sequence-based methods require the isolation of specific populations of immune cells (e.g., T-cells or B-cells), and the spatial isolation of such cells into individual cells and/or individual molecules of nucleic acid derived from such cells to form colonies (see e.g., US2010/0151471). In contrast, the present specification provides methods for profiling immunoglobulin isotypes using sensitive, high-throughput sequencing technology to sequence immunoglobulin isotypes directly from a heterogeneous nucleic acid mixture derived from a heterogeneous population of cells. The detection and quantitation of specific immunoglobulin isotypes within the "noise" or "background" of a heterogeneous cell population and a heterogeneous nucleic acid mixture derived therefrom has never been achieved prior to the instant invention. Specifically, isotype can be determined from a very small sample size, such as a single drop of blood. Thus the methods of the invention differ from conventional methods in that the current method is non-invasive and does not require a trained phlebotomist to draw blood from a patient. Moreover, in contrast to conventional methods the method of the present invention does not require fractionation of the blood.

The methods of the invention generally involve the steps of obtaining a peripheral whole blood sample from a subject, isolating RNA from the peripheral whole blood sample, or fraction thereof (e.g., peripheral blood mononuclear cells), reverse transcribing the isolated RNA using target specific primers to generate immunoglobulin cDNA transcripts, amplifying the immunoglobulin VDJ to Ig constant regions using multiplex PCR techniques, sequencing the amplicons, and analyzing the sequence data. Data analysis includes the steps of extracting Ig constant region sequence for each isotype and comparing the total number of all Ig isotype sequences for a given sample.

Monitoring the immune repertoire of healthy and diseased humans by sampling cells derived from peripheral whole blood (e.g., peripheral blood cells, or peripheral blood mononuclear cells) can reveal disease signatures at the immunoglobulin isotype level. By comparing the amount of each isotype present by sequencing amplified cDNA derived from peripheral blood, it is possible to detect levels such that diseased individuals are distinct from healthy individuals.

### Subjects

The methods of the invention utilize biological samples from subjects or individuals. The subject can be a patient, for example, a patient with an autoimmune disease, an infectious disease or cancer, or a transplant recipient. As specified by the claims, the subject is a human. The subject can be a male or female subject of any age (e.g., a fetus, an infant, a child, or an adult).

### Samples

Samples used in the methods of the provided invention can include, for example, a bodily fluid from a subject, including amniotic fluid surrounding a fetus, aqueous humor, bile, blood and blood plasma, cerumen (earwax), Cowper's fluid or pre-ejaculatory fluid, chyle, chyme, female ejaculate, interstitial fluid, lymph, menses, breast milk, mucus (including snot and phlegm), pleural fluid, pus, saliva, sebum (skin oil), semen, serum, sweat, tears, urine, vaginal lubrication, vomit, feces, internal body fluids including cerebrospinal fluid surrounding the brain and the spinal cord, synovial fluid surrounding bone joints, intracellular fluid (the fluid inside cells), and vitreous humour (the fluids in the eyeball).

In one embodiment, the sample is a blood sample, such as a peripheral whole blood sample, or a fraction thereof. Preferably, the sample is whole, unfractionated blood.

The blood sample can be about 0.02, 0.03, 0.04, 0.05, 0.06, 0.07, 0.08 0.09, 0.1, 0.2, 0.3, 0.4, 0.5, 0.6, 0.7, 0.8, 0.9, 1.0, 1.5, 2.0, 2.5, 3.0, 3.5, 4.0, 4.5, or 5.0 mL. Preferably, the sample is 100ul or less. Most preferably the sample size is 50 uL or less.

In certain embodiments falling within the scope of the invention as specified by the claims, the sample is Cerebral Spinal Fluid (CSF) (e.g., when the subject has multiple sclerosis), synovial fluid (e.g., when the subject has rheumatoid arthritis), or skin (or other organ) biopsy (e.g., when the subject has systemic lupus).

Without intending to be bound by any theory, immunoglobulin isotypes can be identified from the available body fluid/tissue most likely to reflect pathology followed by later monitoring of the levels of the isotypes and clonotypic signatures of a particular disease from a different body fluid, for example, blood.

Samples can be analyzed in accordance with the methods of the invention at a time when the disease is both inactive and active, to help identify a clonotypic signature associated with a particular disease.

The sample can be obtained by a health care provider, for example, a physician, physician assistant, nurse, veterinarian, dermatologist, rheumatologist, dentist, paramedic, or surgeon. The sample can be obtained by a research technician. More than one sample from a subject can be obtained.

The sample can be a biopsy, e.g., a skin biopsy. The biopsy can be from, for example, brain, liver, lung, heart, colon, kidney, or bone marrow. Any biopsy technique used by those skilled in the art can be used for isolating a sample from a subject. For example, a biopsy can be an open biopsy, in which general anesthesia is used. The biopsy can be a closed biopsy, in which a smaller cut is made than in an open biopsy. The biopsy can be a core or incisional biopsy, in which part of the tissue is removed. The biopsy can be an excisional biopsy, in which attempts to remove an entire lesion are made. The biopsy can be a fine needle aspiration biopsy, in which a sample of tissue or fluid is removed with a needle.

The sample includes immune cells, as specified by the claims. The immune cells can include T-cells and B-cells. T-cells (T lymphocytes) include, for example, cells that express T cell receptors. T-cells include Helper T cells (effector T cells or Th cells), cytotoxic T cells (CTLs), memory T cells, and regulatory T cells. The sample can include a single cell in some applications (e.g., a calibration test to define relevant T cells) or more generally at least 1,000, at least 10,000, at least 100,000, at least 250,000, at least 500,000, at least 750,000, or at least 1,000,000 T-cells.

B-cells include, for example, plasma B cells, memory B cells, B1 cells, B2 cells, marginal-zone B cells, and follicular B cells. B-cells can express immunoglobulins (antibodies, B cell receptor). As specified by the claims, the sample includes at least 10,000 B-cells. In some instances, the sample can include at least 100,000, at least 250,000, at least 500,000, at least 750,000, or at least 1,000,000 B-cells.

The sample can include nucleic acid, for example, DNA (e.g., genomic DNA or mitochondrial DNA) or RNA (e.g., messenger RNA or microRNA). The nucleic acid can be cell-free DNA or RNA. In the methods described herein, the amount of RNA or DNA from a subject that can be analyzed includes, for example, as low as a single cell in some applications (e.g., a calibration test) and as many as 10 millions of cells or more translating to a range of DNA of 6 pg-60 ug, and RNA of approximately 1 pg 10 ug.

### Amplification Reactions

Polymerase chain reaction (PCR) can be used to amplify the relevant regions from a collection of cells. Transcription Mediated Amplification (TMA) can be used to produce RNA amplicons from a target nucleic acid. The nucleic acid from each cell can be analyzed separately (e.g., via sequencing analysis) as each cell will carry its own unique immunoglobulin isotype signature.

In some embodiments, the VDJ to Ig constant regions of an immunoglobulin sequence are amplified from heterogeneous nucleic acid using multiplex PCR.

In some embodiments falling within the scope of the invention as specified by the claims, immunoglobulin sequences are amplified from heterogeneous nucleic acid in a multiplex reaction using at least one primer that anneals to the C region and one or more primers that can anneal to one or more V segments. The number of primers that anneal to V segments in a multiplex reaction can be, for example, 10-60, 20-50, 30-50, 40-50, 20-40, 30-40, or 35-40. The primers can anneal to different V segments. For IgH genes, because of the possibility of somatic mutations in the V segments, multiple primers that anneal to each V segment can be used, for example, 1, 2, 3, 4, or 5 primers per V segment. The number of primers that anneal to C segments in a multiplex reaction can include, for example, at least 1, 2, 3, 4, 5, 6, 7, 8, 9, 10, 11, 12, 13, 14, or 15. The number of primers that anneal to C segments in a multiplex reaction can be 1-10, 2-9, 3-8, 4-7, 3-8, or 3-6.

In some embodiments falling within the scope of the invention as specified by the claims, the region to be amplified includes the full clonal sequence or a subset of the clonal sequence, including the V-D junction, D-J junction of an immunoglobulin or T-cell receptor gene, the full variable region of an immunoglobulin or T-cell receptor gene, the antigen recognition region, or a CDR, e.g., complementarity determining region 3 (CDR3).

In some embodiments falling within the scope of the invention as specified by the claims, the immunoglobulin sequence is amplified using a primary and a secondary amplification step. Each of the different amplification steps can comprise different primers. The different primers can introduce sequence not originally present in the immune gene sequence. For example, the amplification procedure can add one or more tags to the 5' and/or 3' end of amplified immunoglobulin sequence. The tag can be a sequence that facilitates subsequent sequencing of the amplified DNA. The tag can be a sequence that facilitates binding the amplified sequence to a solid support. The tag can be a bar-code or label to facilitate identification of the amplified immunoglobulin sequence.

Other methods for amplification may not employ any primers in the V region. Instead, a specific primer can be used from the C segment and a generic primer can be put in the other side (5'). The generic primer can be appended in the cDNA synthesis through different methods including the well described methods of strand switching. Similarly, the generic primer can be appended after cDNA making through different methods including ligation.

Other means of amplifying nucleic acid that can be used in the methods of the invention include, for example, reverse transcription-PCR, real-time PCR, quantitative real-time PCR, digital PCR (dPCR), digital emulsion PCR (dePCR), clonal PCR, amplified fragment length polymorphism PCR (AFLP PCR), allele specific PCR, assembly PCR, asymmetric PCR (in which a great excess of primers for a chosen strand is used), colony PCR, helicase-dependent amplification (HDA), Hot Start PCR, inverse PCR (IPCR), in situ PCR, long PCR (extension of DNA greater than about 5 kilobases), multiplex PCR, nested PCR (uses more than one pair of primers), single-cell PCR, touchdown PCR, loop-mediated isothermal PCR (LAMP), and nucleic acid sequence based amplification (NASBA). Other amplification schemes include: Ligase Chain Reaction, Branch DNA Amplification, Rolling Circle Amplification, Circle to Circle Amplification, SPIA amplification, Target Amplification by Capture and Ligation (TACL) amplification, and RACE amplification.

The information in RNA in a sample can be converted to cDNA by using reverse transcription using techniques well known to those of ordinary skill in the art (see e.g., Sambrook, Fritsch and Maniatis, MOLECULAR CLONING: A LABORATORY MANUAL, 2nd edition (1989)). PolyA primers, random primers, and/or gene specific primers can be used in reverse transcription reactions.

Polymerases that can be used for amplification in the methods of the provided invention include, for example, Taq polymerase, AccuPrime polymerase, or Pfu. The choice of polymerase to use can be based on whether fidelity or efficiency is preferred.

After amplification of DNA from the genome (or amplification of nucleic acid in the form of cDNA by reverse transcribing RNA), the amplicons are directly sequenced.

### Sequencing

Any technique for sequencing nucleic acid known to those skilled in the art can be used in the methods of the provided invention. DNA sequencing techniques include classic dideoxy sequencing reactions (Sanger method) using labeled terminators or primers and gel separation in slab or capillary, sequencing-by-synthesis using reversibly terminated labeled nucleotides, pyrosequencing, 454 sequencing, allele specific hybridization to a library of labeled oligonucleotide probes, sequencing-by-synthesis using allele specific hybridization to a library of labeled clones that is followed by ligation, real time monitoring of the incorporation of labeled nucleotides during a polymerization step, and SOLiD sequencing.

As specified by the claims, the sequencing technique used in the methods of the provided invention generates at least 10,000 reads per run. In some instances, the sequencing technique can generate at least 50,000 reads per run, at least 100,000 reads per run, at least 500,000 reads per run, at least 1,000,000 reads per run, at least 2,000,000 reads per run, at least 3,000,000 reads per run, at least 4,000,000 reads per run, at least 5000,000 reads per run, at least 6,000,000 reads per run, at least 7,000,000 reads per run, at least 8,000,000 reads per run, at least 9,000,000 reads per run, or at least 10,000,000 reads per run.

The number of sequencing reads per B cell sampled can be at least 2 times the number of B cells sampled, at least 3 times the number of B cells sampled, at least 5 times the number of B cells sampled, at least 6 times the number of B cells sampled, at last 7 times the number of B cells sampled, at least 8 times the number of B cells sampled, at least 9 times the number of B cells sampled, or at least at least 10 times the number of B cells sampled but not limited to 5x (fewer or greater may be sufficient). So something like 1 million to 10 million reads per sample. The read depth allows for accurate coverage of B cells sampled, facilitates error correction, and ensures that the sequencing of the library has been saturated.

In certain instances, the sequencing technique used in the methods described herein can generate about 30 bp, about 40 bp, about 50 bp, about 60 bp, about 70 bp, about 80 bp, about 90 bp, about 100 bp, about 110 bp, about 120 bp per read, about 150 bp, about 200 bp, about 250 bp, about 300 bp, about 350 bp, about 400 bp, about 450 bp, about 500 bp, about 550 bp, about 600 bp, about 700 bp, about 800 bp, about 900 bp, or about 1,000 bp per read. For example, the sequencing technique used in the methods described herein can generate at least 30, 40, 50, 60, 70, 80, 90, 100, 110, 120, 150, 200, 250, 300, 350, 400, 450, 500, 550, 600, 650, 700, 750, 800, 850, 900, 950, or 1,000 bp per read.

### True Single Molecule Sequencing

A sequencing technique that can be used in the methods of the provided invention includes, for example, Helicos True Single Molecule Sequencing (tSMS) (Harris T. D. et al. (2008) Science 320:106-109). In the tSMS technique, a DNA sample is cleaved into strands of approximately 100 to 200 nucleotides, and a polyA sequence is added to the 3' end of each DNA strand. Each strand is labeled by the addition of a fluorescently labeled adenosine nucleotide. The DNA strands are then hybridized to a flow cell, which contains millions of oligo-T capture sites that are immobilized to the flow cell surface. The templates can be at a density of about 100 million templates/cm². The flow cell is then loaded into an instrument, e.g., HeliScope™. sequencer, and a laser illuminates the surface of the flow cell, revealing the position of each template. A CCD camera can map the position of the templates on the flow cell surface. The template fluorescent label is then cleaved and washed away. The sequencing reaction begins by introducing a DNA polymerase and a fluorescently labeled nucleotide. The oligo-T nucleic acid serves as a primer. The polymerase incorporates the labeled nucleotides to the primer in a template directed manner. The polymerase and unincorporated nucleotides are removed. The templates that have directed incorporation of the fluorescently labeled nucleotide are detected by imaging the flow cell surface. After imaging, a cleavage step removes the fluorescent label, and the process is repeated with other fluorescently labeled nucleotides until the desired read length is achieved. Sequence information is collected with each nucleotide addition step.

### 454 Sequencing

Another example of a DNA sequencing technique that can be used in the methods of the provided invention is 454 sequencing (Roche) (Margulies, M et al. 2005, Nature, 437, 376-380). 454 sequencing involves two steps. In the first step, DNA is sheared into fragments of approximately 300-800 base pairs, and the fragments are blunt ended. Oligonucleotide adaptors are then ligated to the ends of the fragments. The adaptors serve as primers for amplification and sequencing of the fragments. The fragments can be attached to DNA capture beads, e.g., streptavidin-coated beads using, e.g., Adaptor B, which contains 5'-biotin tag. The fragments attached to the beads are PCR amplified within droplets of an oil-water emulsion. The result is multiple copies of clonally amplified DNA fragments on each bead. In the second step, the beads are captured in wells (pico-liter sized). Pyrosequencing is performed on each DNA fragment in parallel. Addition of one or more nucleotides generates a light signal that is recorded by a CCD camera in a sequencing instrument. The signal strength is proportional to the number of nucleotides incorporated.

Pyrosequencing makes use of pyrophosphate (PPi) which is released upon nucleotide addition. PPi is converted to ATP by ATP sulfurylase in the presence of adenosine 5' phosphosulfate. Luciferase uses ATP to convert luciferin to oxyluciferin, and this reaction generates light that is detected and analyzed.

### Genome Sequencer FLX™

Another example of a DNA sequencing technique that can be used in the methods of the invention is the Genome Sequencer FLX systems (Roche/454). The Genome Sequences FLX systems (e.g., GS FLX/FLX+, GS Junior) offer more than 1 million high-quality reads per run and read lengths of 400 bases. These systems are ideally suited for de novo sequencing of whole genomes and transcriptomes of any size, metagenomic characterization of complex samples, or resequencing studies.

### SOLiD™ Sequencing

Another example of a DNA sequencing technique that can be used in the methods of the provided invention is SOLiD technology (Life Technologies, Inc.). In SOLiD sequencing, genomic DNA is sheared into fragments, and adaptors are attached to the 5' and 3' ends of the fragments to generate a fragment library. Alternatively, internal adaptors can be introduced by ligating adaptors to the 5' and 3' ends of the fragments, circularizing the fragments, digesting the circularized fragment to generate an internal adaptor, and attaching adaptors to the 5' and 3' ends of the resulting fragments to generate a mate-paired library. Next, clonal bead populations are prepared in microreactors containing beads, primers, template, and PCR components. Following PCR, the templates are denatured and beads are enriched to separate the beads with extended templates. Templates on the selected beads are subjected to a 3' modification that permits bonding to a glass slide.

The sequence can be determined by sequential hybridization and ligation of partially random oligonucleotides with a central determined base (or pair of bases) that is identified by a specific fluorophore. After a color is recorded, the ligated oligonucleotide is cleaved and removed and the process is then repeated.

### Ion Torrent™ Sequencing

Another example of a DNA sequencing technique that can be used in the methods of the provided invention is the IonTorrent system (Life Technologies, Inc.). Ion Torrent uses a high-density array of micro-machined wells to perform this biochemical process in a massively parallel way. Each well holds a different DNA template. Beneath the wells is an ion-sensitive layer and beneath that a proprietary Ion sensor. If a nucleotide, for example a C, is added to a DNA template and is then incorporated into a strand of DNA, a hydrogen ion will be released. The charge from that ion will change the pH of the solution, which can be detected by the proprietary ion sensor. The sequencer will call the base, going directly from chemical information to digital information. The Ion Personal Genome Machine (PGM™) sequencer then sequentially floods the chip with one nucleotide after another. If the next nucleotide that floods the chip is not a match, no voltage change will be recorded and no base will be called. If there are two identical bases on the DNA strand, the voltage will be double, and the chip will record two identical bases called. Because this is direct detection-no scanning, no cameras, no light-each nucleotide incorporation is recorded in seconds.

### HiSeq™ and MiSeq™ Sequencing

Additional examples of sequencing technologies that can be used in the methods of the invention include the HiSEQ™ system (e.g., HiSEQ2000™ and HiSEQ1000™) and the MiSEQ™ system from Illumina, Inc. The HiSEQ™ system is based on massively parallel sequencing of millions of fragments using attachment of randomly fragmented genomic DNA to a planar, optically transparent surface and solid phase amplification to create a high density sequencing flow cell with millions of clusters, each containing about 1,000 copies of template per sq. cm. These templates are sequenced using four-color DNA sequencing-by-synthesis technology. The MiSEQ™ system uses TruSeq, Illumina's reversible terminator-based sequencing-by-synthesis.

### SOLEXA™ Sequencing

Another example of a sequencing technology that can be used in the methods of the invention is SOLEXA sequencing (Illumina). SOLEXA sequencing is based on the amplification of DNA on a solid surface using fold-back PCR and anchored primers. Genomic DNA is fragmented, and adapters are added to the 5' and 3' ends of the fragments. DNA fragments that are attached to the surface of flow cell channels are extended and bridge amplified. The fragments become double stranded, and the double stranded molecules are denatured. Multiple cycles of the solid-phase amplification followed by denaturation can create several million clusters of approximately 1,000 copies of single-stranded DNA molecules of the same template in each channel of the flow cell. Primers, DNA polymerase and four fluorophore-labeled, reversibly terminating nucleotides are used to perform sequential sequencing. After nucleotide incorporation, a laser is used to excite the fluorophores, and an image is captured and the identity of the first base is recorded. The 3' terminators and fluorophores from each incorporated base are removed and the incorporation, detection and identification steps are repeated.

### SMRT™ Sequencing

Another example of a sequencing technology that can be used in the methods of the provided invention includes the single molecule, real-time (SMRT™) technology of Pacific Biosciences. In SMRT™, each of the four DNA bases is attached to one of four different fluorescent dyes. These dyes are phospholinked. A single DNA polymerase is immobilized with a single molecule of template single stranded DNA at the bottom of a zero-mode waveguide (ZMW). A ZMW is a confinement structure which enables observation of incorporation of a single nucleotide by DNA polymerase against the background of fluorescent nucleotides that rapidly diffuse in and out of the ZMW (in microseconds). It takes several milliseconds to incorporate a nucleotide into a growing strand. During this time, the fluorescent label is excited and produces a fluorescent signal, and the fluorescent tag is cleaved off. Detection of the corresponding fluorescence of the dye indicates which base was incorporated. The process is repeated.

### Nanopore Sequencing

Another example of a sequencing technique that can be used in the methods of the provided invention is nanopore sequencing (Soni G V and Meller A. (2007) Clin Chem 53: 1996-2001). A nanopore is a small hole, of the order of 1 nanometer in diameter. Immersion of a nanopore in a conducting fluid and application of a potential across it results in a slight electrical current due to conduction of ions through the nanopore. The amount of current which flows is sensitive to the size of the nanopore. As a DNA molecule passes through a nanopore, each nucleotide on the DNA molecule obstructs the nanopore to a different degree. Thus, the change in the current passing through the nanopore as the DNA molecule passes through the nanopore represents a reading of the DNA sequence.

### Chemical-Sensitive Field Effect Transistor Array Sequencing

Another example of a sequencing technique that can be used in the methods of the provided invention involves using a chemical-sensitive field effect transistor (chemFET) array to sequence DNA (for example, as described in US Patent Application Publication No. 20090026082). In one example of the technique, DNA molecules can be placed into reaction chambers, and the template molecules can be hybridized to a sequencing primer bound to a polymerase. Incorporation of one or more triphosphates into a new nucleic acid strand at the 3' end of the sequencing primer can be detected by a change in current by a chemFET. An array can have multiple chemFET sensors. In another example, single nucleic acids can be attached to beads, and the nucleic acids can be amplified on the bead, and the individual beads can be transferred to individual reaction chambers on a chemFET array, with each chamber having a chemFET sensor, and the nucleic acids can be sequenced.

### Sequencing with an Electron Microscope

Another example of a sequencing technique that can be used in the methods of the provided invention involves using an electron microscope (Moudrianakis E. N. and Beer M. Proc Natl Acad Sci USA. 1965 March; 53:564-71). In one example of the technique, individual DNA molecules are labeled using metallic labels that are distinguishable using an electron microscope. These molecules are then stretched on a flat surface and imaged using an electron microscope to measure sequences.

Any one of the sequencing techniques described herein can be used in the methods of the invention.

### Digital Counting and Analysis

Sequencing allows for the presence of multiple immunoglobulin isotypes to be detected and quantified in a heterogeneous biological sample. Sequence data analysis includes the steps of extracting Ig constant region sequence for each isotype and comparing the total number of all Ig isotype sequences for a given sample. High-throughput analysis can be achieved using one or more bioinformatics tools, such as ALLPATHS (a whole genome shotgun assembler that can generate high quality assemblies from short reads), Arachne (a tool for assembling genome sequences from whole genome shotgun reads, mostly in forward and reverse pairs obtained by sequencing cloned ends, BACCardl (a graphical tool for the validation of genomic assemblies, assisting genome finishing and intergenome comparison), CCRaVAT & QuTie (enables analysis of rare variants in large-scale case control and quantitative trait association studies), CNV-seq (a method to detect copy number variation using high throughput sequencing), Elvira (a set of tools/procedures for high throughput assembly of small genomes (e.g., viruses)), Glimmer (a system for finding genes in microbial DNA, especially the genomes of bacteria, archaea and viruses), gnumap (a program designed to accurately map sequence data obtained from next-generation sequencing machines), Goseq (an R library for performing Gene Ontology and other category based tests on RNA-seq data which corrects for selection bias), ICAtools (a set of programs useful for medium to large scale sequencing projects), LOCAS, a program for assembling short reads of second generation sequencing technology, Maq (builds assembly by mapping short reads to reference sequences), MEME (motif-based sequence analysis tools), NGSView (allows for visualization and manipulation of millions of sequences simultaneously on a desktop computer, through a graphical interface), OSLay (Optimal Syntenic Layout of Unfinished Assemblies), Perm (efficient mapping for short sequencing reads with periodic full sensitive spaced seeds), Projector (automatic contig mapping for gap closure purposes), Qpalma (an alignment tool targeted to align spliced reads produced by sequencing platforms such as Illumina, Solexa, or 454), RazerS (fast read mapping with sensitivity control), SHARCGS (SHort read Assembler based on Robust Contig extension for Genome Sequencing; a DNA assembly program designed for *de novo* assembly of 25-40mer input fragments and deep sequence coverage), Tablet (next generation sequence assembly visualization), and Velvet (sequence assembler for very short reads).

### Methods and Uses

The methods disclosed herein are used with subjects at risk for developing a disease or disorder, subjects who may or may not have already been diagnosed with a disease or disorder and subjects undergoing treatment and/or therapies for a disease or disorder. The methods of the present invention can also be used to monitor or select a treatment regimen for a subject who has a disease or disorder, and to screen subjects who have not been previously diagnosed as having a disease or disorder, such as subjects who exhibit risk factors for the disease or disorder. Preferably, the methods of the present invention are used to identify and/or diagnose subjects who are asymptomatic for a disease or disorder. "Asymptomatic" means not exhibiting the traditional symptoms.

The methods of the present invention may also be used to identify and/or diagnose subjects already at higher risk of developing a disease or disorder based on solely on the traditional risk factors.

A subject having a disease or disorder can be identified by determining an isotype profile in a subject-derived sample and the amounts are then compared to a reference value. Alterations in isotype profile in the subject sample compared to the reference value are then identified.

A reference value can be relative to a number or value derived from population studies, including without limitation, such subjects having the same disease or disorder subject having the same or similar age range, subjects in the same or similar ethnic group, subjects having family histories of the disease or disorder, or relative to the starting sample of a subject undergoing treatment for a disease or disorder. Such reference values can be derived from statistical analyses and/or risk prediction data of populations obtained from mathematical algorithms and computed indices of the disease or disorder. Reference isotype profile indices can also be constructed and used using algorithms and other methods of statistical and structural classification.

In one instance of the present invention, the reference profile is the isotype profile in a control sample derived from one or more subjects who are not at risk or at low risk for developing the disease or disorder. In another instance of the present invention, the reference profile is the isotype profile in a control sample derived from one or more subjects who are asymptomatic and/or lack traditional risk factors for a disease or disorder. In a further instance, such subjects are monitored and/or periodically retested for a diagnostically relevant period of time ("longitudinal studies") following such test to verify continued absence of a disease or disorder (disease or event free survival). Such period of time may be one year, two years, two to five years, five years, five to ten years, ten years, or ten or more years from the initial testing date for determination of the reference value. Furthermore, retrospective measurement of isotype profiles in properly banked historical subject samples may be used in establishing these reference values, thus shortening the study time required.

A reference profile can also comprise the isotype profiles derived from subjects who show an improvement in disease or disorder risk factors as a result of treatments and/or therapies for the disease or disorder. A reference profile can also comprise the isotype profiles derived from subjects who have confirmed disease by known invasive or non-invasive techniques, or are at high risk for developing disease or disorder, or who have suffered from a disease or disorder.

In another instance, the reference value is an index value or a baseline value. An index value or baseline value is a composite sample from a normal subject not having the disease. A baseline value can also comprise the isotype profile in a sample derived from a subject who has shown an improvement risk factors as a result of treatments or therapies. In this instance, to make comparisons to the subject-derived sample, the amounts control sample are similarly calculated and compared to the index value.

The progression of a disease or disorder or effectiveness of a disease or disorder treatment regimen can be monitored by detecting an isotype profile in samples obtained from a subject over time and comparing the amount of isotype profiles detected. For example, a first sample can be obtained prior to the subject receiving treatment and one or more subsequent samples are taken after or during treatment of the subject. The disease or disorder is considered to be progressive (or, alternatively, the treatment does not prevent progression) if the isotype profile changes over time relative to the reference value, whereas the disease or disorder is not progressive if the isotype profile remains constant over time (relative to the reference population, or "constant" as used herein). The term "constant" as used in the context of the present invention is construed to include changes over time with respect to the reference value.

Additionally, therapeutic or prophylactic agents suitable for administration to a particular subject can be identified by detecting an isotype profile in a sample obtained from a subject, exposing the subject-derived sample to a test compound. Accordingly, treatments or therapeutic regimens for use in subjects having a disease or disorder, or subjects at risk for developing a disease or disorder can be selected based on the isotype profiles in samples obtained from the subjects and compared to a reference value. Two or more treatments or therapeutic regimens can be evaluated in parallel to determine which treatment or therapeutic regimen would be the most efficacious for use in a subject to delay onset, or slow progression of the disease or disorder.

The present specification further provides a method for screening for changes in isotype profiles with a disease or disorder, by determining the isotype profile in a subject-derived sample, comparing the isotype profile in a reference sample, and identifying alterations in the isotype profile in the subject sample compared to the reference sample.

If the reference sample, e.g., a control sample, is from a subject that does not have a disease or disorder, or if the reference sample reflects a value that is relative to a person that has a high likelihood of rapid progression to a disease or disorder, a similarity in the isotype profile in the test sample and the reference sample indicates that the treatment is efficacious. However, a difference in the isotype profile in the test sample and the reference sample indicates a less favorable clinical outcome or prognosis.

Assessment of the risk factors disclosed herein can be achieved using standard clinical protocols. Efficacy can be determined in association with any known method for diagnosing, identifying, or treating a disease or disorder.

Also described is a method for treating one or more subjects having a disease or disorder by determining the isotype profile in a sample from the one or more subjects; and treating the one or more subjects with one or more drugs until the isotype profile return to a baseline value measured in one or more subjects at low risk for developing a disease or disorder.

Also described is a method for evaluating changes in the risk of developing a disease or disorder in a subject, by isotype profile in a first sample from the subject at a first period of time, determining the isotype profile in a second sample from the subject at a second period of time, and comparing the isotype profiles detected at the first and second periods.

The "normal isotype profile" means a profile typically found in a subject not suffering from a disease or disorder. Such normal control level and cutoff points may vary based on whether an isotype profile is used alone or in a formula combining with other clinical indicators of the disease or disorder into an index. Alternatively, the normal control level can be a database of isotype profiles from previously tested subjects who did not develop a disease or disorder clinically relevant time horizon.

The present invention may be used to make continuous or categorical measurements of the risk of conversion to a disease state, thus diagnosing and defining the risk spectrum of a category of subjects defined as at risk for having a disease state. In the categorical scenario, the methods of the present invention can be used to discriminate between normal and disease subject cohorts. In other embodiments, the present invention may be used so as to discriminate those at risk for having a disease event from those having more rapidly progressing (or alternatively those with a shorter probable time horizon to disease event) to a disease event from those more slowly progressing (or with a longer time horizon to a disease event), or those having a disease from normal.

Identifying the subject at risk of having a disease or disorder enables the selection and initiation of various therapeutic interventions or treatment regimens in order to delay, reduce or prevent that subject's conversion to a disease state. Isotype profiles allows for the course of treatment of a disease to be monitored. In this method, a biological sample can be provided from a subject undergoing treatment regimens, e.g., drug treatments. If desired, biological samples are obtained from the subject at various time points before, during, or after treatment.

The present invention can also be used to screen patient or subject populations in any number of settings. For example, a health maintenance organization, public health entity or school health program can screen a group of subjects to identify those requiring interventions, as described above, or for the collection of epidemiological data. Insurance companies (e.g., health, life or disability) may screen applicants in the process of determining coverage or pricing, or existing clients for possible intervention. Data collected in such population screens, particularly when tied to any clinical progression to conditions like cancer or metastatic events, will be of value in the operations of, for example, health maintenance organizations, public health programs and insurance companies. Such data arrays or collections can be stored in machine-readable media and used in any number of health-related data management systems to provide improved healthcare services, cost effective healthcare, improved insurance operation, etc. See, for example, U.S. Patent Application No. 2002/0038227; U.S. Patent Application No. US 2004/0122296; U.S. Patent Application No. US 2004/0122297; and U.S. Patent No 5,018,067. Such systems can access the data directly from internal data storage or remotely from one or more data storage sites as further detailed herein.

A machine-readable storage medium can comprise a data storage material encoded with machine readable data or data arrays which, when using a machine programmed with instructions for using said data, is capable of use for a variety of purposes, such as, without limitation, subject information relating to metastatic disease risk factors over time or in response drug therapies. Measurements of effective amounts of the biomarkers of the invention and/or the resulting evaluation of risk from those biomarkers can implemented in computer programs executing on programmable computers, comprising, *inter alia*, a processor, a data storage system (including volatile and non-volatile memory and/or storage elements), at least one input device, and at least one output device. Program code can be applied to input data to perform the functions described above and generate output information. The output information can be applied to one or more output devices, according to methods known in the art. The computer may be, for example, a personal computer, microcomputer, or workstation of conventional design.

Each program can be implemented in a high level procedural or object oriented programming language to communicate with a computer system. However, the programs can be implemented in assembly or machine language, if desired. The language can be a compiled or interpreted language. Each such computer program can be stored on a storage media or device (e.g., ROM or magnetic diskette or others as defined elsewhere in this disclosure) readable by a general or special purpose programmable computer, for configuring and operating the computer when the storage media or device is read by the computer to perform the procedures described herein. The health-related data management system described herein may also be considered to be implemented as a computer-readable storage medium, configured with a computer program, where the storage medium so configured causes a computer to operate in a specific and predefined manner to perform various functions described herein.

Isotype profiles are determined and compared to a reference value, e.g. a control subject or population whose disease state is known or an index value or baseline value. The reference sample or index value or baseline value may be taken or derived from one or more subjects who have been exposed to the treatment, or may be taken or derived from one or more subjects who are at low risk of developing a disease or disorder, or may be taken or derived from subjects who have shown improvements in as a result of exposure to treatment. Alternatively, the reference sample or index value or baseline value may be taken or derived from one or more subjects who have not been exposed to the treatment. For example, samples may be collected from subjects who have received initial treatment for a disease or disorder and subsequent treatment for the disease or disorder to monitor the progress of the treatment. A reference value can also comprise a value derived from risk prediction algorithms or computed indices from population studies such as those disclosed herein.

The isotype profiles can be used to generate "reference isotype profile" of those subjects who do not have a disease or disorder or are not at risk of having a disease or a disorder, and would not be expected to develop a disease or disorder. The DETERMINANTS disclosed herein can also be used to generate a "subject isotype profile" taken from subjects who have cancer or are at risk for a disease or disorder. The subject isotype profiles can be compared to a reference isotype profile to diagnose or identify subjects at risk for developing a disease or disorder to monitor the progression of disease, as well as the rate of progression of disease, and to monitor the effectiveness of treatment modalities. The reference and subject isotype profiles of the present specification can be contained in a machine-readable medium, such as but not limited to, analog tapes like those readable by a VCR, CD-ROM, DVD-ROM, USB flash media, among others. Such machine-readable media can also contain additional test results, such as, without limitation, measurements of clinical parameters and traditional laboratory risk factors. Alternatively or additionally, the machine-readable media can also comprise subject information such as medical history and any relevant family history. The machine-readable media can also contain information relating to other disease-risk algorithms and computed indices such as those described herein.

### Definitions

*"Accuracy"* refers to the degree of conformity of a measured or calculated quantity (a test reported value) to its actual (or true) value. Clinical accuracy relates to the proportion of true outcomes (true positives (TP) or true negatives (TN)) versus misclassified outcomes (false positives (FP) or false negatives (FN)), and may be stated as a sensitivity, specificity, positive predictive values (PPV) or negative predictive values (NPV), or as a likelihood, odds ratio, among other measures.

A "*baseline profile data set"* is a set of values associated with constituents of a *Gene Expression Panel* (Precision Profile™) resulting from evaluation of a biological sample (or population or set of samples) under a desired *biological condition* that is used for mathematically normative purposes. The desired biological condition may be, for example, the condition of a subject (or population or set of subjects) before exposure to an agent or in the presence of an untreated disease or in the absence of a disease. Alternatively, or in addition, the desired biological condition may be health of a subject or a population or set of subjects. Alternatively, or in addition, the desired biological condition may be that associated with a population or set of subjects selected on the basis of at least one of age group, gender, ethnicity, geographic location, nutritional history, medical condition, clinical indicator, medication, physical activity, body mass, and environmental exposure.

*"FN"* is false negative, which for a disease state test means classifying a disease subject incorrectly as non-disease or normal.

*"FP"* is false positive, which for a disease state test means classifying a normal subject incorrectly as having disease.

*A "formula," "algorithm,"* or *"model"* is any mathematical equation, algorithmic, analytical or programmed process, statistical technique, or comparison, that takes one or more continuous or categorical inputs (herein called *"parameters")* and calculates an output value, sometimes referred to as an "*index*" or "*index value."* Non-limiting examples of *"formulas"* include comparisons to reference values or profiles, sums, ratios, and regression operators, such as coefficients or exponents, value transformations and normalizations (including, without limitation, those normalization schemes based on clinical parameters, such as gender, age, or ethnicity), rules and guidelines, statistical classification models, and neural networks trained on historical populations. In panel and combination construction, of particular interest are structural and synactic statistical classification algorithms, and methods of risk index construction, utilizing pattern recognition features, including, without limitation, such established techniques such as cross-correlation, Principal Components Analysis (PCA), factor rotation, Logistic Regression Analysis (LogReg), Kolmogorov Smirnoff tests (KS), Linear Discriminant Analysis (LDA), Eigengene Linear Discriminant Analysis (ELDA), Support Vector Machines (SVM), Random Forest (RF), Recursive Partitioning Tree (RPART), as well as other related decision tree classification techniques (CART, LART, LARTree, FlexTree, amongst others), Shrunken Centroids (SC), StepAIC, K-means, Kth-Nearest Neighbor, Boosting, Decision Trees, Neural Networks, Bayesian Networks, Support Vector Machines, and Hidden Markov Models, among others. Other techniques may be used in survival and time to event hazard analysis, including Cox, Weibull, Kaplan-Meier and Greenwood models well known to those of skill in the art. Many of these techniques are useful either combined with a constituent of a Gene Expression Panel (Precision Profile™) selection technique, such as forward selection, backwards selection, or stepwise selection, complete enumeration of all potential panels of a given size, genetic algorithms, voting and committee methods, or they may themselves include biomarker selection methodologies in their own technique. These may be coupled with information criteria, such as Akaike's Information Criterion (AIC) or Bayes Information Criterion (BIC), in order to quantify the tradeoff between additional biomarkers and model improvement, and to aid in minimizing overfit. The resulting predictive models may be validated in other clinical studies, or cross-validated within the study they were originally trained in, using such techniques as Bootstrap, Leave-One-Out (LOO) and 10-Fold cross-validation (10-Fold CV). At various steps, false discovery rates (FDR) may be estimated by value permutation according to techniques known in the art.

*"Index"* is an arithmetically or mathematically derived numerical characteristic developed for aid in simplifying or disclosing or informing the analysis of more complex quantitative information. A disease or population index may be determined by the application of a specific algorithm to a plurality of subjects or samples with a common biological condition.

*"Negative predictive value"* or *"NPV"* is calculated by TN/(TN + FN) or the true negative fraction of all negative test results. It also is inherently impacted by the prevalence of the disease and pre-test probability of the population intended to be tested.

See, *e.g.,* O'Marcaigh AS, Jacobson RM, "Estimating the Predictive Value of a Diagnostic Test, How to Prevent Misleading or Confusing Results," Clin. Ped. 1993, 32(8): 485-491, which discusses specificity, sensitivity, and positive and negative predictive values of a test, *e.g.,* a clinical diagnostic test. Often, for binary disease state classification approaches using a continuous diagnostic test measurement, the sensitivity and specificity is summarized by Receiver Operating Characteristics (ROC) curves according to Pepe et al., "Limitations of the Odds Ratio in Gauging the Performance of a Diagnostic, Prognostic, or Screening Marker," Am. J. Epidemiol 2004, 159 (9): 882-890, and summarized by the Area Under the Curve (AUC) or c-statistic, an indicator that allows representation of the sensitivity and specificity of a test, assay, or method over the entire range of test (or assay) cut points with just a single value. See also, e.g., Shultz, "Clinical Interpretation Of Laboratory Procedures," chapter 14 in Teitz, Fundamentals of Clinical Chemistry, Burtis and Ashwood (eds.), 4th edition 1996, W.B. Saunders Company, pages 192-199; and Zweig et al., "ROC Curve Analysis: An Example Showing the Relationships Among Serum Lipid and Apolipoprotein Concentrations in Identifying Subjects with Coronary Artery Disease," Clin. Chem., 1992, 38(8): 1425-1428. An alternative approach using likelihood functions, BIC, odds ratios, information theory, predictive values, calibration (including goodness-of-fit), and reclassification measurements is summarized according to Cook, "Use and Misuse of the Receiver Operating Characteristic Curve in Risk Prediction," Circulation 2007, 115: 928-935.

*"Positive predictive value"* or *"PPV"* is calculated by TP/(TP+FP) or the true positive fraction of all positive test results. It is inherently impacted by the prevalence of the disease and pre-test probability of the population intended to be tested.

*"Risk"* in the context of the present invention, relates to the probability that an event will occur over a specific time period, and can mean a subject's "absolute" risk or "relative" risk. Absolute risk can be measured with reference to either actual observation post-measurement for the relevant time cohort, or with reference to index values developed from statistically valid historical cohorts that have been followed for the relevant time period. Relative risk refers to the ratio of absolute risks of a subject compared either to the absolute risks of lower risk cohorts, across population divisions (such as tertiles, quartiles, quintiles, or deciles, etc.) or an average population risk, which can vary by how clinical risk factors are assessed. Odds ratios, the proportion of positive events to negative events for a given test result, are also commonly used (odds are according to the formula p/(1-p) where p is the probability of event and (1- p) is the probability of no event) to no-conversion.

*"Risk evaluation*," or "*evaluation of risk"* in the context of the present invention encompasses making a prediction of the probability, odds, or likelihood that an event or disease state may occur, and/or the rate of occurrence of the event or conversion from one disease state to another, *i.e.,* from a normal condition to cancer or from cancer remission to cancer, or from primary cancer occurrence to occurrence of a cancer metastasis. Risk evaluation can also comprise prediction of future clinical parameters, traditional laboratory risk factor values, or other indices of cancer results, either in absolute or relative terms in reference to a previously measured population. Such differing use may require different constituents of a Gene Expression Panel (Precision Profile™) combinations and individualized panels, mathematical algorithms, and/or cut-off points, but be subject to the same aforementioned measurements of accuracy and performance for the respective intended use.

"*Sensitivity*" is calculated by TP/(TP+FN) or the true positive fraction of disease subjects.

"*Specificity*" is calculated by TN/(TN+FP) or the true negative fraction of non-disease or normal subjects.

By *"statistically, significant",* it is meant that the alteration is greater than what might be expected to happen by chance alone (which could be a "false positive"). Statistical significance can be determined by any method known in the art. Commonly used measures of significance include the *p*-value, which presents the probability of obtaining a result at least as extreme as a given data point, assuming the data point was the result of chance alone. A result is often considered highly significant at a *p*-value of 0.05 or less and statistically significant at a *p*-value of 0.10 or less. Such *p*-values depend significantly on the power of the study performed.

*"TN"* is true negative, which for a disease state test means classifying a non-disease or normal subject correctly.

*"TP"* is true positive, which for a disease state test means correctly classifying a disease subject.

The invention having now been described by way of written description, those of skill in the art will recognize that the invention can be practiced in a variety of embodiments and that the foregoing and examples below are for purposes of illustration and not limitation of the claims that follow.

### EXAMPLES

The following examples, including the experiments conducted and results achieved are provided for illustrative purposes only and are not to be construed as limiting upon the present invention.

### Example 1: Isotype results obtained by sequencing amplified cDNA

The present invention is based, in part, upon isotype distribution patterns that were noted by the inventors when comparing isotype distribution data in studies regarding normal patients vs. patients who received the influenza vaccine. Earlier data (not shown) revealed IgM to be in greater amount for naïve B cells isolated from patients prior to an influenza vaccine. Post vaccine, plasma B cells showed the IgG was the most abundant isotype.

Based on this observation, the following study was designed to analyze the isotype distribution pattern in normal patients vs. patients suffering from SLE.

Methods: Briefly, PBMCs were isolated and stored in DMSO under liquid nitrogen. Samples were quickly thawed and rapidly washed in phosphate buffered saline prior to total RNA extraction. Total RNA was reverse transcribed using target specific primers to generate immunoglobulin cDNA transcripts. Multiplex PCR was used to amplify immunoglobulin VDJ to Ig constant regions. Amplicons were prepared for sequencing and sequenced using 454 Sequencing (Roche). Data analysis included extracting Ig constant region sequence for each isotype and comparing to the total number of all Ig isotype sequences for a given sample.

In the isotype distribution data of normal vs. SLE patients, no cell sorting was done, however the results showed a predominance of IgM for the normal patient (naïve B cells predominate as the system is in immune monitoring mode). For the SLE patients, again no cell sorting was done, but the results showed a predominance of IgG, indicating a higher level of active plasma B cells. The results are shown in Figure 1 (normal sample (#1) was dominated by IgM, typical of Naïve B cells. Samples #2 and #3, taken from patients burdened with SLE, were dominated by IgG, typical of Plasma B cells responding to environmental stress). Averages over runs and disease type are provided in the right hand figures. This result was consistent with the SLE samples being taken during a flare, for example, where one would expect to see many B cells converting to plasma B cells in order to produce IgG.

Ratios of IgM to IgG indicate whether an individual is in survey mode or mounting an immune response. The signal for the SLE patient showed increased IgG and IgA isotypes while IgM and IgD isotypes were decreased as compared to the normal subjects. The level of IgM observed from these PBMCs was consistent with the 60% reported in the literature for healthy adults (see references below).

The foregoing embodiments are to be considered in all respects illustrative rather than limiting on the invention described herein.

## Claims

1. A method of determining whether an immunoglobulin isotype profile determined from a human subject-derived biological sample is indicative of a disease state, comprising:
a. isolating and amplifying a plurality of RNA nucleic acids from the biological sample, wherein the RNA comprises Ig isotype constant region sequences, to generate amplicons that comprise immunoglobulin constant region sequences, wherein the biological sample comprises a plurality of different cell types including at least 10,000 B-cells;
b. sequencing the amplicons in a massively parallel sequencing reaction using a sequencing technique that generates at least 10,000 sequence reads per run;
c. analyzing sequence data from (b) by extracting Ig constant region sequences characteristic of each isotype, and comparing the number of isotype sequences for each isotype thereby determining the immunoglobulin isotype profile; and
d. determining whether the immunoglobulin isotype profile is characteristic of a disease state, wherein the disease state is an autoimmune disease, an infectious disease, or cancer.

2. The method of claim 1, wherein the disease state is systemic lupus erythmatosus (SLE).

3. The method of claim 1 or 2, wherein the amplifying comprises obtaining cDNA from the RNA prior to step (b).

4. The method of claim 2, wherein the immunoglobulin isotype profile characteristic of a subject with SLE is increased in IgG and IgA isotypes and decreased in IgM and IgD isotypes compared to the immunoglobulin isotype profile characteristic of a healthy adult.

5. The method of any of claims 1-4, wherein the amplifying in step (a) comprises a multiplex amplification reaction using 1-10 primers that hybridize to Ig constant region sequences and 10-60 primers that hybridize to Ig variable region sequences.

6. The method of claims 1-5, wherein the biological sample is blood or a fraction thereof.

7. The method of claim 6, wherein the blood biological sample size is 100 µL or less.

8. The method of claim 6 or 7, wherein the blood is peripheral whole blood.

9. The method of claim 6 or 7, wherein the biological sample is a blood fraction comprising peripheral blood mononuclear cells.

10. The method of any of claims 1-9, wherein the massively parallel sequencing reaction is a sequencing-by-synthesis reaction.

11. The method of claim 10, wherein the sequence by synthesis reaction comprises single molecule sequencing.

12. The method of any of claims 1-11, wherein the amplicons comprise the immunoglobulin VDJ region.

## Patentansprüche

1. Verfahren zum Ermitteln, ob ein Immunglobulinisotypprofil, das aus einer von einem menschlichen Patienten abgeleiteten biologischen Probe ermittelt wurde, für einen Krankheitszustand bezeichnend ist, umfassend:
a. Isolieren und Amplifizieren einer Vielzahl von RNA-Nukleinsäuren aus der biologischen Probe, wobei die RNA Sequenzen der konstanten Region des Ig-Isotyps umfasst, um Amplicons zu erzeugen, die Sequenzen der konstanten Region von Immunglobulin umfassen, wobei die biologische Probe eine Vielzahl unterschiedlicher Zelltypen umfasst, einschließend mindestens 10.000 B-Zellen;
b. Sequenzieren der Amplicons in einer massiv parallelen Sequenzierungsreaktion unter Verwendung einer Sequenzierungsmethode, die pro Durchgang mindestens 10.000 Sequenzablesungen erzeugt;
c. Analysieren von Sequenzdaten von (b), indem Sequenzen der konstanten Region von Ig extrahiert werden, die für jeden Isotyp charakteristisch sind und Vergleichen der Anzahl von Isotypsequenzen für jeden Isotyp, wodurch das Immunglobulinisotypprofil ermittelt wird; und
d. Ermitteln, ob das Immunglobulinisotypprofil für einen Krankheitszustand charakteristisch ist, wobei der Krankheitszustand eine Autoimmunkrankheit, eine Infektionskrankheit oder Krebs ist.

2. Verfahren nach Anspruch 1, wobei der Krankheitszustand systemischer Lupus erythematodes (SLE) ist.

3. Verfahren nach Anspruch 1 oder 2, wobei das Amplifizieren das Gewinnen von cDNA aus der RNA vor dem Schritt (b) umfasst.

4. Verfahren nach Anspruch 2, wobei das Immunglobulinisotypprofil, das für einen Patienten mit SLE charakteristisch ist, im Vergleich zu dem Immunglobulinisotypprofil, das für einen gesunden Erwachsenen charakteristisch ist, in IgG- und IgA-Isotypen erhöht und in IgM- und IgD-Isotypen verringert ist.

5. Verfahren nach einem der Ansprüche 1-4, wobei das Amplifizieren in Schritt (a) eine Multiplexamplifikationsreaktion unter Verwendung von 1-10 Primern, die sich an Sequenzen der konstanten Region von Ig hybridisieren und 10-60 Primern umfasst, die sich an Sequenzen der variablen Region von Ig hybridisieren.

6. Verfahren nach den Ansprüchen 1-5, wobei die biologische Probe Blut oder eine Fraktion davon ist.

7. Verfahren nach Anspruch 6, wobei die Größe der biologischen Blutprobe 100 µL oder weniger beträgt.

8. Verfahren nach Anspruch 6 oder 7, wobei das Blut peripheres Vollblut ist.

9. Verfahren nach den Ansprüchen 6 oder 7, wobei die biologische Probe eine Blutfraktion ist, umfassend mononukleäre Zellen des peripheren Bluts.

10. Verfahren nach einem der Ansprüche 1-9, wobei die massiv parallele Sequenzierungsreaktion eine Sequenzierung-durch-Synthese-Reaktion ist.

11. Verfahren nach Anspruch 10, wobei die Sequenz-durch-Synthese-Reaktion die Sequenzierung einzelner Moleküle umfasst.

12. Verfahren nach einem der Ansprüche 1-11, wobei die Amplicons die Immunglobulin-VDJ-Region umfassen.

## Revendications

1. Procédé permettant de déterminer si un profil d'isotype d'immunoglobuline déterminé à partir d'un échantillon biologique provenant d'un sujet humain est indicateur d'un état pathologique, consistant à :
a. isoler et amplifier une pluralité d'acides nucléiques d'ARN provenant de l'échantillon biologique, ledit ARN comprenant des séquences de région constante d'isotype d'Ig, pour générer des amplicons qui comprennent des séquences de région constante d'immunoglobuline, ledit échantillon biologique comprenant une pluralité de différents types de cellules dont au moins 10000 lymphocytes B ;
b. séquencer des amplicons dans une réaction de séquençage massivement parallèle au moyen d'une technique de séquençage qui génère au moins 10000 lectures de séquences par cycle ;
c. analyser des données séquentielles de (b) par extraction de séquences de région constante d'Ig de chaque isotype, et la comparaison du nombre de séquences d'isotype pour chaque isotype, déterminant ainsi le profil d'isotype d'immunoglobuline ; et
d. déterminer si le profil d'isotype d'immunoglobuline est caractéristique d'un état pathologique, ledit état pathologique étant une maladie auto-immune, une maladie infectieuse ou un cancer.

2. Procédé selon la revendication 1, ledit état pathologique étant le lupus érythémateux systémique (LES).

3. Procédé selon la revendication 1 ou 2, ladite amplification comprenant l'obtention d'ADNc à partir de l'ARN avant l'étape (b).

4. Procédé selon la revendication 2, ledit profil d'isotype d'immunoglobuline caractéristique d'un sujet atteint de LES étant accru dans les isotypes d'IgG et d'IgA et diminué dans les isotypes d'IgM et d'IgD par rapport au profil d'isotype d'immunoglobuline caractéristique d'un adulte en bonne santé.

5. Procédé selon l'une quelconque des revendications 1 à 4, ladite étape d'amplification dans l'étape (a) comprenant une réaction d'amplification multiplex au moyen de 1 à 10 amorces qui s'hybrident aux séquences de région constante d'Ig et de 10 à 60 amorces qui s'hybrident à des séquences de région variable d'Ig.

6. Procédé selon les revendications 1 à 5, ledit échantillon biologique étant du sang ou une fraction de celui-ci.

7. Procédé selon la revendication 6, la taille dudit échantillon biologique de sang étant inférieure ou égale à 100 µl.

8. Procédé selon la revendication 6 ou 7, le sang étant du sang périphérique entier.

9. Procédé selon la revendication 6 ou 7, ledit échantillon biologique étant une fraction de sang comprenant des cellules mononucléaires de sang périphérique.

10. Procédé selon l'une quelconque des revendications 1 à 9, ladite réaction de séquençage massivement parallèle étant une réaction de séquençage par synthèse.

11. Procédé selon la revendication 10, ladite réaction de séquençage par synthèse comprenant le séquençage de molécule unique.

12. Procédé selon l'une quelconque des revendications 1 à 11, lesdits amplicons comprenant la région VDJ d'immunoglobuline.
